Europäisches Patentamt

European Patent Office

Office européen des brevets

(19)

(11) Publication number: **0 285 405**
A1

(12)

# EUROPEAN PATENT APPLICATION

(21) Application number: 88302862.3

(22) Date of filing: 30.03.88

(51) Int. Cl.4: **C 12 N 15/00**
C 12 P 21/02, C 07 K 13/00
// A61K37/64

(30) Priority: 31.03.87 US 32859

(43) Date of publication of application:
05.10.88 Bulletin 88/40

(84) Designated Contracting States: ES GR

(71) Applicant: Schering Biotech Corporation
901 California Avenue
Palo Alto California 94304-1104 (US)

THE JOHNS HOPKINS UNIVERSITY
720 Rutland Avenue
Baltimore, MD 21205 (US)

(72) Inventor: Ishizaka, Kimishige
618 Lake Drive
Towson Maryland 21204 (US)

Martens, Christine L.
141 Erica Way
Menlo Park California 94025 (US)

Moore, Kevin W.
4144 Park Boulevard
Palo Alto California 94306 (US)

(74) Representative: Ritter, Stephen David et al
Mathys & Squire 10 Fleet Street
London EC4Y 1AY (GB)

(54) Glycosylation inhibition factors.

(57) cDNA clones are provided which allow the production of polypeptides exhibiting glycosylation inhibiting factor (GIF) activity. Such polypeptides are useful in the treatment of atopic disease. A clone, designated TGIF4, which is capable of encoding mouse polypeptides having GIF activity, is deposited with the ATCC under accession number 67311.

FIGURE 1

**Description**

## GLYCOSYLATION INHIBITING FACTORS

The invention relates generally to immunosuppressive proteins, and more particularly to polypeptides exhibiting glycosylation-inhibiting factor (GIF) activity and nucleotide sequences encoding such polypeptides.

As far as can be determined, the main physiological function of IgE-mediated responses is to combat parasites. The response can be divided into five phases: an IgE-bearing B cell is stimulated to respond to an antigen (phase 1) and activated to secrete IgE antibodies (phase 2); the antibodies produced bind to mast cells and basophils in tissues (phase 3, antibody fixation); interaction of allergen with cell-bound IgE activates these cells and causes the release of chemical mediators stored in their granules (phase 4, degranulation); and finally the mediators induce a complex tissue response aimed at the elimination of nonmicrobial parasites from the body (phase 5). Part of this defense mechanism is an attack on the tissue that harbors the parasite--that is, on self. To excise a parasite from a tissue without damaging the rest of the body is an extraordinarily delicate act. The mediators released by activated mast cells and basophils can cause considerable harm, even death, if released at an inappropriate time or if directed at an inappropriate target. The IgE response must be closely controlled and quickly attenuated after its goal has been achieved. As long as this control is functioning there is no danger that healthy parts of the body will be damaged but, should the controls fail, the beneficial reaction will turn into a harmful one. In humans, about 90 percent of all individuals have no difficulty in using their IgE only for defensive purposes; but the remaining unlucky 10 percent carry a genetic defect of the control mechanism that permits the stimulation of IgE responses by antigens that have nothing to do with parasites. At first it was thought that this defect was limited to humans, but similar defects were discovered later in several other mammals. The inappropriately stimulated IgE responses cause a plethora of diverse diseases, grouped under the name allergy or atopy; see Klein, Immunology: The Science of Self- Nonself Discrimination (John Wiley &; Sons, New York, 1982).

Currently glucocorticoid steroids are the most effective drugs for treating allergic diseases. However, prolonged steroid treatment is associated with many deleterious side effects: Goodman and Gillman, The Pharmacological Basis of Therapeutics, 6th ed. (MacMillan Publishing Company, New York, 1980). Recently, Ishizaka and his co-workers have discovered and characterized a class of compounds which they have designated as glycosylation inhibiting factors (GIFs): Ishizaka, Ann. Rev. Immunol., Vol. 2, pgs. 159-182 (1984). GIFs are natural compounds which are capable of causing T cells to produce a class of IgE binding factors which, in turn, selectively suppress the IgE response (IgE suppressive factors). Apparently, one GIF is related to lipomodulin, a phospholipase inhibitory protein, which is induced by treatment of a variety of cells with glucocorticoids, e.g. Flower et al., Nature, Vol. 278, pgs 456-459 (1979). This GIF apparently inhibits the assembly or processing of N-linked oligosaccharide on IgE binding factors resulting in the selective formation of IgE suppressive factors. Moreover, it suppresses IgE-induced expression of Fc-epsilon receptors on lymphocytes; it has a molecular weight of 15-16 kilodaltons; and it binds to monoclonal antibodies against a lipomodulin: Uede et al., J. Immunol., Vol. 130, pgs 878-884 (1983), and Iwata et al., J. Immunol., Vol. 132, pgs. 1286-1294 (1984).

Lipomodulins, also referred to in the literature as lipocortins, are agents which are believed to inhibit the activity of phospholipase $A_2$, an enzyme involved in the synthesis of inflammation-inducing compounds, such as prostaglandins and leukotrienes. It has been shown that alkaline-phosphatase-treated GIF is capable of inhibiting phospholipase $A_2$, suggesting that GIF may be a phosphorylated lipomodulin: Uede et al. (cited above); Ishizaka (cited above).

More recently, Wallner et al., in Nature, Vol. 320, pgs. 77-81 (1986), and in PCT Patent Application WO 86/04094 (17 July 1986), reported the cloning, sequencing, and expression of a human lipocortin complementary DNA (cDNA).

In view of the potential clinical utility of lipocortins and GIFs as anti-inflammatory agents, it would be advantageous to provide new methods for their production and new compounds having similar or identical biological properties.

The present invention is directed to mammalian glycosylation-inhibiting factors (GIFs) which are capable of suppressing IgE immunoglobulin responses. The invention includes nucleic acids capable of encoding polypeptides exhibiting GIF activity, and methods employing such nucleic acids to produce GIF polypeptides. GIF polypeptides of the invention are encoded by the nucleic acid whose sequence is disclosed below in Formula I, or by nucleic acids effectively homologous to the nucleic acid of Formula I.

```
CGT - CAC - TCA - GTA - CCT - ATT - TCA -
                30
AGC - CTT - CCT - GGG - CCC - CCG - AGA -
                                    60
TAC - AAG - AGT - CAA - AGG - ATG - GTT -

CCT - CCT - GGA - CAA - TTA - CAT - CCC -
 90
TAC - GTT - TGT - CTG - TTC - TGT - TAT -
                            120
TTA - CTT - ACT - CAT - TGT - ATG - GCT -

GGG - ACC - AAA - ATA - CAT - GAA - GAA -
150
CCG - GCA - GCC - GTT - CTC - TTG - CCG -
                      180
AGC - ATC - CTG - CAG - TTG - TAT - AAC -
                                    210
CTT - GGA - CTC - ACC - CTG - CTG - TCT -

CTC - TAC - ATG - TTC - TAT - GAG - TTG -
                240
GTG - ACA - GGT - GTG - TGG - GAA - GGC -
                                270
AAA - TAC - AAC - TTT - TTC - TGC - CAG -

GGA - ACA - CGC - AGC - GCG - GGA - GAA -
      300
TCC - GAT - ATG - AAG - ATC - ATC - CGC -
                            330
GTC - CTC - TGG - TGG - TAC - TAC - TTC -

TCC - AAA - CTC - ATC - GAA - TTC - ATG -
360
GAC - ACC - TTT - TTC - TTC - ATC - CTT -
                390
CGC - AAG - AAC - AAC - CAC - CAG - ATC -
                                    420
ACC - GTG - CTC - CAT - GTC - TAC - CAC -

CAC - GCT - ACC - ATG - CTC - AAC - ATC -
            450
TGG - TGG - TTT - GTG - ATG - AAC - TGG -
```

```
                                        480
GTT - CCC - TGC - GGC - CAT - TCA - TAT -

TTT - GGT - GCG - ACA - CTC - AAC - AGC -
      510
TTC - ATC - CAT - GTC - CTC - ATG - TAC -
                        540
TCG - TAC - TAT - GGT - CTG - TCC - TCC -

ATC - CCG - TCC - ATG - CGT - CCC - TAC -
570
CTC - TGG - TGG - AAA - AAG - TAC - ATC -
                  600
ACT - CAA - GGG - CAG - CTG - GTC - CAG -
                                    630
TTT - GTG - CTG - ACA - ATC - ATC - CAG -

ACG - ACC - TGC - GGG - GTC - TTC - TGG -
            660
CCA - TGC - TCC - TTC - CCT - CTC - GGG -
                              690
TGG - CTG - TTC - TTC - CAG - ATT - GGA -

TAC - ATG - ATT - TCC - CTG - ATT - GCT -
      720
CTC - TTC - ACA - AAC - TTC - TAC - ATT -
                        750
CAG - ACT - TAC - AAC - AAG - AAA - GGG -

GCC - TCT - CGG - AGG - AAA - GAA - CAC -
780
CTG - AAG - GGC - CAC - CAG - AAC - GGG -
                  810
TCT - GTG - GCC - GCC - GTC - AAC - GGA -
                                    840
CAC - ACC - AAC - AGC - TTC - CCT - TCC -

CTG - GAA - AAC - AGC - GTG - AAG - CCC -
            870
AGG - AAG - CAG - CGA - AAG - GAT - TGA -
                              900
CAA - GCC - GAG - CCG - AAG - CCT - CCA -

GAC - CGC - GGC - GTG - TGA - TTG - TAA -
      930
GCA - CAG - CGT - GAC - TTG - TGC - CCT -
                        960
GAC - GCT - CAT - AGC - AGC - TGC - TGT -

CAC - TAG - TCT - GGC - CCT - ACT - ATC -
990
TGC - AGT - GTG - ACT - CAT - GTA - GGA -
```

1020

```
CCT - TCT - TCA - TCA - GTT - CAA - AAC -
                                              1050
CCC - TAG - AAT - ATG - TAT - ACA - GAT -

TAT - ACA - AAG - TAG - GCA - TAC - GAT -
            1080
TTT - CTC - ACA - TTT - CTG - GGC - TCT -
                                        1110
CAT - CGA - CCC - CCG - CGC - TAG - ACT -

GTG - GAA - AGG - GAA - TGT - TCT - TGT -
      1140
AGT - ATA - CAA - CAC - TGC - TGT - TGC -
                        1170
CTT - ATT - AGT - TGT - AAC - ACG - ATA -

GGT - GCT - GAA - TTG - TGG - TTC - ACA -
1200
ATT - TAA - AAC - ACT - GTA - ATC - CAA -
                  1230
ACT - TTT - TTT - TTT - TAA - CTG - TAG -
                                    1260
ACC - ATG - CAT - GTG - ATT - GTA - AAT -

TTG - TAC - AAT - GTT - GTT - AAC - AGT -
            1290
AGA - AAA - ACA - CAC - ATG - CCT - TAA -
                              1320
GAC - TTA - AAA - AAG - CAG - GGC - CCA -

GAG - CTT - GTT - AGT - TTA - ATT - AGG -
      1350
GTA - TGT - TTC - AAC - TTT - GTA - TTA -
                  1380
TTC - ATT - TGT - AAT - AGC - TCT - GTT -

TAG - AAA - AAT - CAA - ATG - TAT - GAT -
1410
TTA - TGA - AAC - AAG - TGT - GTG - ATG -
                  1440
TGT - AAC - TTC - AAG - TGA - CTT - GGA -
                              1470
GAT - GCG - AAA - TCT - GAT - GGC - ACC -

CTT - GGT - TTT - ATA - ACG - ATG - GCT -
            1500
TTA - AAT - AAG - CAG - GCT - CAA - GTC -
                              1530
TAG - TGG - AAC - AGT - CAG - TTT - AAC -

TTT - TTA - ACA - GAT - CTT - ATT - TTT -
```

```
TTA - TTT - TGA - GTG - CCA - CTA - TTA -

ATG - TGT - TTA - AAG - AGG - TGG - GGA -

AAG - GGT - CCA - AAG - CGT - CTG - GAG -

ACA - CTT - ACG - TAC - CTG - TCC - CTG -

GTC - CCC - CAG - AGG - CTA - GGG - TGA -

CTA - TAA - GAG - TAG - GTC - ACT - TTT -

CAC - TTC - TGA - AGT - GCT - TAG - TGT -

TTT - TAT - ACA - GGA - GAT - AAA - AAG -

GCC - ATT - CGA - CTT - CCC - ACC - ACT -

TGG - AGA - GAG - AGA - ACT - ACC - TTT -

CAG - GAA - AGG - TGA - CGA - GAC - ATT -

TAT - TAT - CTG - ACA - GAC - ACA - CAC -

ACA - CAC - ACA - CAC - AGT - GAT - AAG -

GGA - CTG - GTT - ACC - TGG - GTC - CAC -

ATT - TTT - TTT - TAT - TCT - TTT - CTG -

TAG - TGC - TAC - TTT - GAC - ATA - CAC -

TTC - CTT - TTA - AAA - TGT - GCC - TAA -

TTT - ACC - AAG - AGA - CCA - GGA - AGC -

ACC - ATA - AGC - ATT - TTG - AGG - TAA -

AAT - TTA - AGA - GCT - GGA - GCG - CGC -

AGG - AAC - TGG - GTG - TTT - GGG - AGC -

AAC - CGC - TTT - TTT - CAG - TGT - GAA -

TCA - TGG - TGG - CAT - CTG - TAA - TTT -

TTT - TCT - GAT - TAG - CAC - CTA - AAG -

ATC - GGT - TTA - AAG - TGC - TTG - GAC -

TGC - TTA - TCC - TAA - AAT - CGT - GTC -
```

```
TAG - TGC - GCT - CGA - GGC - ATC - GGT -
            2130
CAT - ACA - AAT - GGG - GAC - TCT - TTT -
                              2160
CAT - GTG - TGT - CAT - TTT - TAA - AGT -

GTT - GAA - AGC - TTA - GCC - TTT - GAA -
      2190
TAC - CTT - CTG - CAT - TGG - TAA - AAA -
                  2220
ATC - CAT - GGA - ACC - ACT - CAT - GGC -

ACA - TCT - TAG - GAG - ATG - ATT - GAC -
2250
AAT - GTA - TAA - ACT - AAT - TGT - GGG -
                  2280
TTT - GAT - ATT - TAT - GTA - AAT - ACC -
                                    2310
AGT - TTA - CCA - TGC - TTT - AAT - TTT -

GCA - CAT - TCA - TAT - TAC - AAG - GAG -
            2340
CCA - GTT - GGT - CCT - AAT - TAG - CCT -
                              2370
TGT - GGG - TTT - TCT - GTT - TGG - AAG -

GAG - TTG - CGG - CAT - CTG - TTT - ACA -
      2400
TTT - CCC - TTG - TCA - GAT - CTA - GAA -
                  2430
TGT - GTG - TAT - GTG - TGT - GTC - TTC -

TCC - CTA - GGT - ACC - CGT - CTG - CAG -
      2460
GTG - TCT - TTA - CAT - ATC - TCA - ATA -
                  2490
CAA - AAA - TTA - TAA - CAT - TTG - ATA -

GTG - TGC - TGT - CAA - AGT - GTG - CTT -
2520
AGC - TCA - TCT - GGA - TAT - ACC - CAC -
                  2550
ACT - GTT - AAA - TAA - TGT - CTA - AAC -
                              2580
ATT - AAT - CAT - TAA - AAC - CTT - TTT -

GAT - TAA - AAA
```

<u>Formula I</u>

A pcD plasmid designated TGIF4 carrying a cDNA insert having the sequence of Formula I is deposited with the American Type Culture Collection (Rockville, Maryland, USA) under accession number 67311. GIF activity as the term is used herein means that a polypeptide exhibits at least one of the following biological activities, whose assays are described more fully below: (a) IgE binding factor (IgE-BF) switching activity, (b) inhibition of IgE-induced Fc-epsilon receptor expression, and (c) inhibition of phospholipase $A_2$ activity after dephosphorylation.

The invention also includes fragments of the nucleotide sequence disclosed in Formula I which are capable of being used as hybridization probes for screening cDNA or genomic libraries to obtain additional nucleic acids encoding GIFs. Hybridization probes constructed from fragments of the nucleotide sequence of Formula I can range in size from about 18-20 bases to the full length of the disclosed sequence. The hybridization probes of the invention can be synthesized using the sequence information from Formula I, or they can be constructed from restriction endonuclease fragments of the deposited clone TGIF4, whose insert corresponds to the nucleotide sequence of Formula I.

The term "effectively homologous" as used herein means that the nucleotide sequence is capable of being detected by a hybridization probe derived from a cDNA clone of the invention. The exact numerical measure of homology necessary to detect nucleic acids coding for GIF activity depends on several factors including (1) the homology of the probe to non-GIF coding sequences associated with the target nucleic acids, (2) the stringency of the hybridization conditions, (3) whether single-stranded or double-stranded probes are employed, (4) whether RNA or DNA probes are employed, (5) the measures taken to reduce nonspecific binding of the probe, (6) the nature of the method used to label the probe, (7) the fraction of guanidine and cytosine bases in the probe, (8) the distribution of mismatches between probe and target, (9) the size of the probe, and the like.

Preferably, an effectively homologous probe derived from the cDNA of the invention is at least seventy-five to eighty percent (75-80%) homologous to the sequence to be isolated. And most preferably, the effectively homologous probe is at least ninety percent (90%) homologous to the sequence to be isolated.

Homology as the term is used herein is a measure of similarity between two nucleotide (or amino acid) sequences. Homology is expressed as the fraction or percentage of matching bases (or amino acids) after two sequences (possibly of unequal length) have been aligned. The term alignment is used in the sense defined by Sankoff and Kruskal in chapter one of Time Warps, String Edits, and Macromolecules: The Theory and Practice of Sequence Comparison (Addison-Wesley, Reading, MA, 1983). Roughly, two sequences are aligned by maximizing the number of matching bases (or amino acids) between the two sequences with the insertion of a minimal number of "blank" or "null" bases into either sequence to bring about the maximum overlap. Given two sequences, algorithms are available for computing their homology, e.g. Needleham and Wunsch, J. Mol. Biol., Vol. 48, pgs. 443-453 (1970), and Sankoff and Kruskal (cited above) pgs. 23-29. Also, commercial services and software packages are available for performing such comparisons, e.g. Intelligenetics, Inc. (Palo Alto, CA), and University of Wisconsin Genetics Computer Group (Madison, Wisconsin).

The invention will now be described in conjunction with the illustrative Drawings, wherein

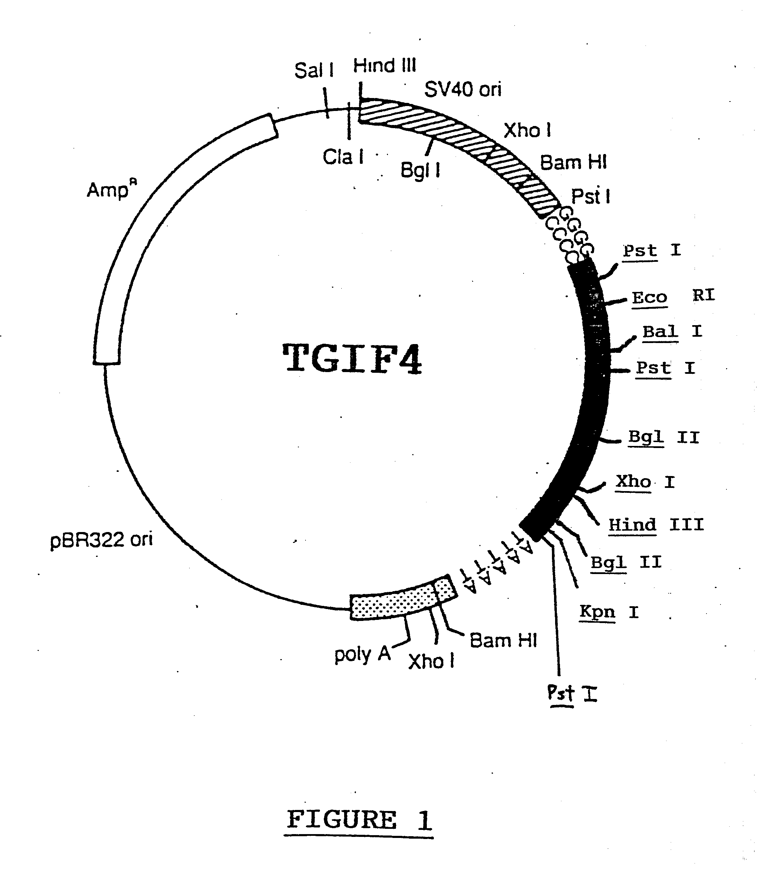

Figure 1 diagrammatically illustrates the pcD plasmid TGIF4.

Techniques for making, using, and identifying the polypeptides and nucleic acids of the invention are discussed below in general terms. Afterward several specific examples are provided wherein the general techniques are applied using specific cell types, vectors, reagents, and the like.

I. De Novo Preparation of GIF cDNA

A variety of methods are now available for de novo preparation and cloning of cDNAs and for the construction of cDNA libraries; e.g. recent reviews are given by Doherty, "Cloning and Expression of cDNA", Chapter in Gottesman, Ed. Molecular Cell Genetics (John Wiley &; Sons, New York, 1985); and Brandis et al., "Preparation of cDNA Libraries and the Detection of Specific Gene Sequences", in Setlow et al., Eds. Genetic Engineering, Vol. 8, pgs. 299-316 (Plenum Press, New York, 1986).

The extraction of total mRNA, its conversion into double-stranded cDNA, the cloning of the cDNA and the transformation of suitable hosts are carried out as outlined in the paragraph bridging pages 30 and 31 of Application PCT/US 86/02464, published as WO 87/02990.

A preferred source of mRNA encoding the desired polypeptides is cells whose supernatants contain one of the activities associated with the polypeptides of the present invention, such as T lymphocytes. RNAs for deriving GIF cDNAs can be obtained from the rat-mouse T hybridoma cell line 23B6 (ATCC accession number HB8521), the human macrophage cell line U-937 (ATCC accession number CRL 1593), and the human promyelocytic cell line HL-60 (ATCC accession number CCL 240). In general, suitable T cells can be obtained from a variety of sources, such as mammalian (e.g. human) spleen, tonsils and peripheral blood. T cell clones, such as those isolated from peripheral blood T-lymphocytes, may also be used (see Research Monographs in Immunology, eds. von Doehmer, H. and Haaf, V.; Section D: "Human T Cell Clones", vol.8, pgs. 243-333; Elsevier Science Publishers, N.Y. [1985]).

If the mRNAs coding for a desired GIF make up a very small fraction of the total mRNA it may be necessary to enrich the fractional concentration in order to make practical the screening procedure for detecting cDNA clones of interest. Appropriate procedures are standard in the art and are disclosed in several papers and references such as Maniatis et al., Molecular Cloning: A Laboratory Manual, Cold Spring Harbor Laboratory, N.Y. 1982 (see pgs. 225-228), Suggs et al., Proc Natl. Acad. Sci., Vol. 78, pgs. 6613-6617 (1981), Parnes et al., Proc. Natl. Acad. Sci., Vol. 78, pgs. 2253-2257 (1981), Davis et al., Proc. Natl. Acad. Sci., Vol. 81, pgs. 2194-2198 (1984) or the like.

A much preferred method of de novo preparation of GIF cDNAs relies on functional expression of the cDNAs in the pcD expression system developed by Okayama and Berg ("functional cloning"), disclosed in Mol. Cell. Biol., Vol. 2, pgs. 161-170 (1982); and Vol. 3. pgs. 280-289 (1983), and available from Pharmacia (Piscataway, N.J.). The pcD expression vector contains the SV40 early promoter, late splicing junction, and the replication origin. This vector permits expression of cDNA inserts in COS 7 monkey cells which provide T antigen for replication of the pcD plasmid. Screening of cDNA libraries includes transfection of pools of plasmid DNA into COS 7 cells using DEAE-Dextran. Since lymphokines are frequently secreted proteins, the supernatants from the transfected cells can be assayed for biological activity after incubation for several days. Positive pools are further divided to identify single cDNA clones which give biological activity after transfection.

Construction of the Okayama and Berg expression vector is briefly described (as for a pcD cDNA library) in Application PCT/US 86/02464 in the paragraph bridging pages 31 and 32.

Once the cDNA library in the Okayama/Berg plasmid vector has been completed, the cDNA clones are collected, and random pools are checked for the presence of the desired cDNAs by standard procedures, e.g. hybrid selection, detection of antigenic determinants on expressed products, and/or functional assays. Positive pools can then be probed with a cDNA from an induced T cell line. Thereafter, the positive, probed pools are divided into individual clones which are further tested by transfection into a suitable host (such as mammalian cells in culture), and the host supernatant is assayed for activity.

## II. Preparation of GIF cDNAs Via Hybridization Probes Derived from Disclosed cDNAs

The cDNAs disclosed herein can be used as probes to identify homologous sequences in different cell types, as an alternative to de novo isolation and cloning of the GIF coding nucleotides. Standard techniques are employed, e.g. Beltz et al., "Isolation of Multigene Families and Determination of Homologies by Filter Hybridization Methods," Methods in Enzymology, Vol. 100, pgs. 266-285 (1983), and Callahan et al., "Detection and Cloning of Human DNA Sequences Related to the Mouse Mammary Tumor Virus Genome," Proc. Natl. Acad. Sci., Vol. 79, pgs. 5503-5507 (1982). Restriction endonuclease fragments of the vector carrying the cDNAs of the invention are used to construct probes (using standard techniques such as nick-translation, e.g. see Maniatis et al., cited above) for screening genomic or cDNA libraries at low hybridization stringencies (again, constructed by standard techniques) of a cell type suspected of producing GIF. Standard screening procedures are employed, e.g. Grunstein et al., Proc. Natl. Acad. Sci., Vol. 72, pgs. 3961-3965 (1975), or Benton et al., Science, Vol. 196, pgs. 180-183 (1977), or Woo, Methods in Enzymology, Vol. 68, pgs. 389-396 (1979). Alternatively, libraries can be screened with labeled oligonucleotide probes whose sequences are determined from Formula I or from the corresponding anti-sense sequence. Such probes can be synthesized on commercially available DNA synthesizers, e.g. Applied Biosystems model 381A, using standard techniques, e.g. Gait Oligonucleotide Synthesis: A Practical Approach, (IRL Press, Washington D.C., 1984). In either case, it is preferable that the probe be at least 18-30 bases long. More preferably, the probe is at least 100-200 bases long. As described below, the BamHI fragment of TGIF4 (see Figure 1) containing the GIF cDNA is a suitable probe for screening for homologous nucleic acids.

Hybridization probes can also be used to screen candidate sources of GIF mRNA prior to library construction.

## III. Preparation of Mutant GIFs by Protein Engineering

General techniques for mutating DNA and producing muteins are described in Application PCT/US 86/02464 (mentioned above) at page 33, line 4 from the bottom to page 34, line 11; these can also be applied in the present invention.

## IV. Assays for Biological Activity.

GIF is assayed (1) by its ability to switch T cells from producing IgE binding factors having potentiating activity (IgE-PF) to producing IgE binding factors having suppressive activity (IgE-SF), (2) by its ability to inhibit IgE-induced expression of Fc-epsilon receptors on lymphocytes, or (3) by its ability, after dephosphorylation, to inhibit phospholipase $A_2$ activity.

### A. IgE-BF Switching Activity.

IgE-BFs are glycoprotein factors produced by T lymphocytes which have affinity for IgE and which regulate the magnitude of IgE responses. At least two forms have been identified: one (IgE-PF) which potentiates the IgE response, and another (IgE-SF) which suppresses the IgE response. In mice IgE-PF and IgE-SF correspond to differently glycosylated forms of the same polypeptide, Martens et al., Proc. Natl. Acad. Sci., Vol. 82, pgs. 2460-2464 (1985) and Vol. 84, pgs. 809-813 (1987). It is believed that the same relationship holds for human IgE-BFs; see Sarfati et al., Immunology, Vol. 53, pgs. 783-790 (1984). Several assays for GIF have been described in the literature which are based on the ability of GIF to cause a switch in the production of IgE-PF to the production of IgE-SF; e.g. Iwata et al., J. Immunol., Vol. 132, pgs. 1286-1293 (1984); Uede et al., J. Immunol., Vol. 130, pgs. 878-884 (1983); Akasaki et al., J. Immunol., Vol. 136, pgs. 3172-3179 (1986); and Jardieu et al., J. Immunol., Vol. 133, pgs. 3266-3273 (1984).

Assays applicable to rodent GIFs are described below. Analogous assays for other mammalian species can be applied in a straightforward manner. Although the assays disclosed by the above publications are for mouse or rat GIFs, it is believed that the biological effects of GIFs are interspecific. For example, rodent GIF from 23B6 cells (deposited with the American Type Culture Collection, Rockville, MD, under accession number HB8521) has been shown to switch human T cell hybridoma 166A2 (described by Huff and Ishizaka in Proc. Natl. Acad. Sci., Vol. 81, pg. 1514 [1984]) from the production of IgE-PF to the production of IgE-SF. In any case, as described more fully below, the analogous human IgE-BF switching assays are readily constructed.

One method for assaying the ability of GIF to switch T cells from IgE-PF to IgE-SF production depends on the observation that the differences between IgE-PF and IgE-SF reside in their respective states of glycosylation, and that these differences can be detected by affinity for certain lectins: Martens et al., Proc. Natl. Acad. Sci., Vol. 82, pgs. 2460-2464 (1985), and Huff et al., Proc. Natl. Acad. Sci., Vol. 81, pgs. 1514-1518 (1984). IgE-PF has affinity for lentil lectin and concanavalin A (ConA), whereas IgE-SF fails to bind to either lectin; see Yodoi et al., J. Immunol., Vol. 128, pg. 289 (1982). Thus, the degree of IgE-specific rosetting by cells known to have Fc-epsilon receptors in the presence of a sample suspected of containing IgE-BFs is an inverse measure of the amount of IgE-BFs in the sample. That is, the IgE-BFs compete with the receptors for binding to IgE bound to microspheres or fixed erythrocytes. For example, normal BALB/c spleen cells can be used to assay GIF activity as follows. Samples of normal spleen cells (about $1 \times 10^7$ nucleated cells/ml) are cultured for about 24 hours with 10 micrograms/ml mouse IgE in the presence or absence (controls) of the GIF samples to be tested, and the IgE binding factors formed in the cultures are fractionated on a lentil lectin Sepharose column. When normal BALB/c spleen cells are cultured with IgE alone, IgE binding factors formed by the cells are distributed approximately equally between the effluent and eluate fractions. The majority of IgE binding factors formed in the presence of a GIF should fail to bind to the lentil lectin Sepharose. Similarly, human GIF can be assayed using BALB/c spleen cells or by substituting human IgE for mouse IgE and a human T cell hybridoma, such as 166A2, or a human cell for BALB/c spleen cells.

IgE binding factors in either the effluent or eluate fractions are measured by their ability to inhibit rosette formation between cells having Fc-epsilon receptors and IgE-coated erythrocytes or microspheres. Erythrocytes coated with human serum albumin (HSA) are used to indicate the level of nonspecific rosettes. IgE-coated erythrocytes are prepared by the method disclosed by Gonzalez-Molina et al., J. Clin. Invest., Vol. 59, pg. 616 (1977), with slight modification disclosed by Yodoi and Ishizaka, J. Immunol., Vol. 122, pg. 2579 (1979). Briefly, ox erythrocytes (Colorado Serum Co., Denver, Colo.), are treated successively with trypsin (Miles Laboratories, Erkhart, Ind.), pyruvic aldehyde (ICN Pharmaceutical Inc., Plainview, N.Y.), and formaldehyde, and the fixed cells are kept in phosphate-buffered saline (PBS), pH 7.2. Sensitization of fixed erythrocytes with human IgE, rat IgE, or human serum albumin (HSA, 2 × crystallized, Nutritional Biochemicals, Cleveland, Ohio) is carried out in 0.1 M acetate buffer, pH 5.0. A 4% suspension of fixed erythrocytes is mixed with an equal volume of a protein solution of an appropriate concentration, and the suspensions are rotated for 2 hr at room temperature. An optimal concentration of each protein for sensitization is determined by preliminary experiments. Preferably, about 0.2 mg/ml of human IgE, 0.25 mg/ml of rat IgE, and 0.25 mg/ml of HSA is employed to sensitize the cells. A 1% suspension of the sensitized cells in PBS is stored at 4°C, and the cells are used for rosette formation within 1 week after coupling.

Mesenteric lymph node (MLN) cells from a rat (e.g. Lewis strain, available from Microbiological Associates) infected with the nematode Nippostrongylus brasiliensis (Nb) can be the source of Fc-epsilon positive cells for the assay. For example, a rat can be infected with 2800-3000 larvae of Nb via a subcutaneous route as described by Ogilvie in Nature, Vol. 204, pg. 91 (1964). MLN cells are obtained 2-3 weeks after infection using standard procedures, e.g. Ishizaka, et al., Cell. Immunol., Vol. 22, pg. 248 (1976). The RPMI 8866 cell line is useful as a source of Fc-epsilon receptor-positive cells in the human GIF assay; e.g. Ishizaka and Sandberg, J. Immunol., Vol. 126, pgs. 1692-1696 (1981).

The rosette inhibition assay proceeds as follows. About 20 microliters of suspended MLN cells (RPMI 8866 for the human assay) (about $5 \times 10^6$/ml) are incubated for about 10 minutes at 37°C with an equal volume of a 1% suspension of fixed erythrocytes coated with IgE (or HSA). The cell suspension is then centrifuged at about 90 × G for 5 minutes, and kept at 0°C for 90 minutes. Pellets are gently mixed with 0.1% toluidine blue in PBS, and then examined in a hemocytometer. At least 300, and preferably 600-1000, cells are counted for enumerating the percentage of rosette-forming cells (RFCs). A positive RFC is defined as a cell having at least three fixed erythrocytes adherent to its surface. HSA-coated fixed erythrocytes are used to quantify the degree of nonspecific rosette formation.

Alternatively, the IgE-BFs can be assayed by the microtiter plate assay disclosed by Hudak and Kehry, in J. Immunol. Methods, Vol. 84, pgs. 11-24 (1985), or by the flow cytometric technique disclosed by Bonnefoy et al. in J. Immunol. Methods, Vol. 88, pgs. 25-32 (1986).

GIF can also be assayed by direct tests of the IgE-SF activity of the binding factors produced in its presence. A source of binding factors is T cells primed with an antigen known to preferentially elicit an IgE response, e.g. dinitrophenylated ovalbumin (DNP-OA) or T cells from atopic patients, e.g. Ishizaka and Sandberg (cited above). (Thus, the IgE-BFs from these sources are presumed to have IgE-PF activity, which in the presence of GIF will be switched to IgE-SF activity.) As mentioned below, an alternative source of IgE-PFs for mouse assays is supernatants from COS7 cells transfected with the plasmid 23B6p8.3, described by Martens et al. (cited above). The suppressive effects of the binding factors are tested on cells which have been induced to produce IgE. In a rodent system, cultures of DNP-OA primed rat mesenteric lymph node (MLN) cells serve as a source of IgE producing cells. The MLN cells are suspended in Click's medium (Cell Immunol., Vol. 3, pg. 264 [1972]) supplemented with 10 percent normal rat serum, 5 × 10⁻⁵M mercaptoethanol, 100 U/ml of penicillin, and 100 micrograms/ml streptomycin. $10^6$/ml of the cells and 0.1 microgram/ml DNP-OA are cultured in microtiter plates. The suppressive effect on the IgE producing cells is assayed in the presence of IgE-PF (i.e., the assay measures the ability of the suspected IgE-SF to counteract the effects of a known IgE-PF). A 24 hour culture filtrate of MLN cells obtained from rats on the 14th day of Nippostrongylus brasiliensis infection can be employed as a source of IgE-PF. An alternative source of IgE-PFs is supernatants of COS7 cells which have been transfected with the plasmid 23B6p8.3, described by Martens et al. (cited above) and deposited with the ATCC under accession number 39633. Both the sample to be tested for suppressive effect and the culture filtrate containing IgE-PF are added to the DNP-OA primed cells. After 5 days of culturing with DNP-OA, the numbers of IgE-containing cells and $IgG_2$-containing cells developed in the culture are compared with those developed in control cultures that contained only DNP-OA and IgE-PF. Ig expression is determined by immunofluorescence, e.g. via labeled rabbit or goat anti-rat IgE or $IgG_2$ antibodies.

B. Inhibition of Fc-epsilon Receptor Expression.

The Fc-epsilon receptor inhibition assay is based on the observation that lymphocytes exposed to IgE express a larger number of Fc-epsilon receptors than they would in the absence of IgE, and that GIF has the property of counteracting or inhibiting such an increase in Fc-epsilon receptors. Thus, the assay requires a source of Fc-epsilon positive lymphocytes which can be cultured in the presence of IgE alone or in combination with a sample suspected of containing GIF. For rodent GIF, rat MLN cells prepared as described above are suitable. After removal from the rat, the cells are dispersed and a portion are cultured in the presence of IgE (control). Other portions are cultured in the presence of IgE and GIF-containing samples. After about 24 hours the MLN cells are tested for their ability to form rosettes with IgE-coated microspheres or fixed erythrocytes. The degree to which rosetting is inhibited relative to the control is a measure of GIF activity. In the rat MLN cell assay the maximum change in rosetting is typically a drop from 30% of the cells (of the control population) forming rosettes to about 15% of the GIF-exposed cells forming rosettes. The human assay can be conducted in a similar manner, also using MLN cells of rats infected with Nb. As in the case of the IgE-BF switching assays the microtiter plate and flow cytometric methods described by Hudak and Kehry (cited above) and Bonnefoy et al. (cited above) can replace rosette counting in the measurement of Fc-epsilon receptor inhibition.

C. Inhibition of Phospholipase $A_2$ Activity by Dephosphorylated GIF.

The dephosphorylation of GIF and the measurement of phospholipase $A_2$ inhibition are carried out by standard assays, e.g. Hirata et al., Proc. Natl. Acad. Sci., Vol. 78, pgs. 3190-3194 (1984), Rothhut et al., Biochem. Biophys. Res. Commun., Vol. 117, pgs. 878-884 (1983), Hirata et al., Proc. Natl. Acad. Sci., Vol. 77, pgs. 2533-2536 (1980), Pepinsky et al., J. Biol. Chem., Vol. 261, pgs. 4239-4246 (1986), Cloix et al., Br. J. Pharmac., Vol. 79, pgs. 313-321 (1983), and Akasaki et al., J. Immunol., Vol. 136, pgs. 3172-3179 (1986).

By way of example, a suitable assay for use with the invention is conducted as follows. One milliliter of a GIF preparation is dialyzed against Tris.HCl buffer, pH 8.2, and is incubated with 0.3 U of insoluble alkaline phosphatase (calf intestinal; Sigma Chemical Co., St. Louis, MO) for 2 hr at room temperature in order to dephosphorylate the GIF. After centrifugation, the supernatant is dialyzed against 0.1 M Tris.HCl buffer, pH 8.0. A solution of 2-¹⁴C-arachidonyl phosphatidylcholine (55 Ci/mol; New England Nuclear, Boston, MA) is dried under $N_2$ and is suspended in 0.1 M Tris-HCl buffer, pH 8.0, by sonication at a concentration of 5 µCi/ml. The reaction mixture consists of 20 µl of an appropriate dilution of a GIF preparation, 0.7 U of porcine pancreatic phospholipase $A_2$ (Sigma Chemical Co.), and 20 µl of radioactive substrate in a total vol of 50 µl. Control tubes contain either phospholipase and the substrate (A), or the radioactive substrate alone (B). After incubation at 37° C for 5 min. the reaction is terminated by the addition of 1 ml Dole's reagent (Gatt and Barenholz, Methods in Enzymology, Vol. 14, pg. 167 (1969)). Distilled water (0.5 ml) and 0.5 ml of heptane are added to the mixture, and [¹⁴C] arachidonic acid hydrolyzed from the β position of phosphatidylcholine is extracted into heptane. To 0.5 ml of the upper phase, 1.0 ml of heptane and silicic acid are added. After mixing, the tubes are centrifuged, and the radioactivity of a 1-ml sample (heptane phase) is measured in a scintillation spectrometer. The percent inhibition of phospholipase is calculated by the following formula:

$$\left(1 - \frac{X - B}{A - B}\right) \times 100$$

in which X represents radioactivity of extracted materials derived from a test mixture, and A and B represent radioactivity from two control tubes.

V. Purification and Pharmaceutical Compositions.

The polypeptides of the present invention, expressed preferably in mammalian (or insect) cells, can be purified according to standard procedures of the art, including ammonium sulfate precipitation, fractionation column chromatography (e.g., ion exchange, gel filtration, electrophoresis, affinity chromatography, etc.) and

11

ultimately crystallization (see generally "Enzyme Purification and Related Techniques", Methods in Enzymology, 22:233-577 [1977] and Scopes, R., Protein Purification: Principles and Practice, Springer-Verlag, New York [1982]).

Pharmaceutical compositions containing the polypeptides of this invention can be formulated and administered substantially as described in Application PCT/US 86/02464 from page 44 line 27 to page 45 line 21. A unit dose according to the present invention preferably contains from 1μg to 100 mg of active polypeptide, according to its potency and application.

VI. Expression Systems

Any appropriate expression system can be chosen; its nature is not critical to the present invention, given that it is appropriate. Suitable expression systems are discussed in Section VI, Expression Systems, on pages 45-50 of Application PCT/US 86/02464.

EXAMPLES

The following examples serve to illustrate the present invention. Selection of vectors and hosts as well as the concentration of reagents, temperatures, and the values of other variables are only to exemplify application of the present invention and are not to be considered limitations thereof.

Example I. Construction of a cDNA Library from 23B6 Cells, Isolation of Clone TGIF4, and Expression of Mouse GIF in COS7 Monkey Cells.

cDNA clones encoding polypeptides exhibiting GIF activity were isolated from a cDNA library constructed from mRNA isolated from IgE-induced 23B6 cells, which were formed by fusion of rat lymphocytes with cells of the AKR mouse thymoma BW 5147. 23B6 is deposited with the American Type Culture Collection under accession number HB 2581. Briefly, a cDNA library was constructed in the pcD vector in accordance with the method of Okayama and Berg (references cited above). The pcD vectors carrying cDNA inserts were amplified in E. coli. pcD-transfected E. coli were plated on agar and individual colonies were randomly picked and amplified. Plasmid DNA was extracted from pools of these randomly picked clones and used to transfect COS 7 monkey cells. The supernatants of the COS 7 cultures were then tested for GIF activity. Such random screening continued until a clone was selected that produced GIF activity. The initially selected GIF cDNA clone was then used as a probe to screen a new pcD cDNA library (again constructed from mRNA isolated from IgE-induced 23B6 cells) in order to select the full sized cDNA clone, which was designated TGIF4.

A. Isolation of mRNA

Total cellular DNA was isolated from 23B6 cells (which produce GIF constitutively) using the guanidine isothiocyanate procedure of Chirgwin, J. et al. (Biochemistry, 18:5294-5299 [1979]). Frozen cell pellets were suspended in guanidine isothiocyanate lysis solution. Twenty ml of lysis solution was used for $1.5 \times 10^8$ cells. Pellets were resuspended by pipetting and then DNA was sheared by 4 passes through a syringe using a 16 gauge needle. The lysate was layered on top of 14 ml of 5.7 M CsCl, 10 mM EDTA in 40 ml polyallomer centrifuge tube. This solution was centrifuged at 25,000 rpm in Beckman SW28 rotor (Beckman Instruments, Inc., Palo Alto, CA) for about 24 hrs at 15°C. The guanidine isothiocyanate phase containing DNA was pipetted off from the top, down to the interface. The walls of the tube and interface were washed with 2-3 ml of guanidine isothiocyanate lysis solution. The tube was cut below the interface with a razor blade, and the CsCl solution was decanted. RNA pellets were washed twice with cold 70% ethanol. Pellets were then resuspended in 500 μl of 10 mM Tris.HCl (pH 7.4), 1 mM EDTA, 0.05% SDS. 50 μl of 3M sodium acetate was added and RNA was precipitated with 1 ml ethanol. About 0.3 mg total RNA was collected by centrifuging and the pellets washed once with cold ethanol.

Washed and dried total RNA pellet was resuspended in 900 μl of oligo (dT) elution buffer (10 mM Tris.HCl, pH 7.4, 1 mM EDTA, 0.1% SDS). RNA was heated for 3 min. at 68°C and then chilled on ice. 100 μl of 5 M NaCl was added. The RNA sample was loaded onto a 1.0 ml oligo (dT) cellulose column (Type 3, Collaborative Research, Waltham, MA) equilibrated with binding buffer (10 mM Tris.HCl pH 7.4, 1 mM EDTA, 0.5 M NaCl, 0.5% SDS.) Flow-through from the column was passed over the column twice more. The column was then washed with 20 ml binding buffer. Poly A⁺ mRNA was collected by washing with elution buffer. RNA usually eluted in the first 2 ml of elution buffer. RNA was precipitated with 0.1 volume 3 M sodium acetate (pH 6) and two volumes of ethanol. The RNA pellet was collected by centrifugation, washed twice with cold ethanol, and dried. The pellet was then resuspended in water. Aliquots were diluted, and absorbance at 260 nm was determined.

B. Construction of the pcD cDNA Library

1) Preparation of Vector Primer and Oligo(dG)-Tailed Linker DNAs.

The procedure of Okayama &; Berg (Mol. &; Cell. Biol. Vol. 2, pgs.161-170 [1982]) was used with only minor modifications. The pcDV1 and pL1 plasmids are described by Okayama &; Berg (Mol. &; Cell. Biol. 3:380-389 [1983]) and are available from Pharmacia (Piscataway, N.J.).

An 80 μg sample of pcDV1 DNA was digested at 30°C with 20 U of KpnI endonuclease in a reaction mixture of 450 μl containing 6 mM Tris.HCl (pH 7.5), 6 mM MgCl₂, 6 mM NaCl, 6 mM 2-ME, and 0.1 mg of bovine serum albumin (BSA) per ml. After 16 hr the digestion was terminated with 40 µl of 0.25 M EDTA (pH 8.0) and 20 µl of 10% sodium dodecyl sulfate (SDS); the DNA was recovered after extraction with water-saturated 1:1 phenol-CHCl₃ (hereafter referred to as phenol-CHCl₃) and precipitation with ethanol. Homopolymer tails averaging 60, but not more than 80, deoxythymidylate (dT) residues per end were added to the KpnI-endonuclease-generated termini with calf thymus terminal transferase as follows: The reaction mixture (38 µl) contained sodium cacodylate-30 mM Tris.HCl (pH 6.8) as buffer, with 1 mM CoCl2, 0.1 mM dithiothreitol, 0.25 mM dTTP, the KpnI-endonuclease-digested DNA, and 68 U of the terminal deoxynucleotidyl transferase (P-L Biochemicals, Inc., Milwaukee, WI). After 30 min. at 37°C the reaction was stopped with 20 µl of 0.25 M EDTA (pH 8.0) and 10 µl of 10% SDS, and the DNA was recovered after several extractions with phenol-CHCl₃ by precipitation with ethanol. The DNA was then digested with 15 U of EcoRI endonuclease in 50 µl containing 10 mM Tris.HCl (pH 7.4), 10 mM MgCl2, 1 mM dithiothreitol, and 0.1 mg of BSA per ml for 5 hr at 37°C. The large fragment, containing the SV40 polyadenylation site and the pBR322 origin of replication and ampicillin-resistance gene, was purified by agarose (1%) gel electrophoresis and recovered from the gel by a modification of the glass powder method (Vogelstein, B. &; Gillespie, D., Proc. Natl. Acad. Sci. 76: 615-619 [1979]). The dT-tailed DNA was further purified by absorption and elution from an oligo (dA)-cellulose column as follows: The DNA was dissolved in 1 ml of 10 mM Tris.HCl (pH 7.3) buffer containing 1 mM EDTA and 1 M NaCl, cooled at 0°C, and applied to an oligo (dA)-cellulose column (0.6 by 2.5 cm) equilibrated with the same buffer at 0°C and eluted with water at room temperature. The eluted DNA was precipitated with ethanol and dissolved in 10 mM Tris.HCl (pH 7.3) with 1 mM EDTA.

The oligo (dG) tailed linked DNA was prepared by digesting 75 µg of pL1 DNA with 20 U of PstI endonuclease in 450 µl containing 6 mM Tris.HCl (pH 7.4), 6 mM MgCl2, 6 mM 2-ME, 50 mM NaCl, and 0.01 mg of BSA per ml. After 16 hr at 30°C the reaction mixture was extracted with phenol-CHCl₃ and the DNA was precipitated with ethanol. Tails of 10 to 15 deoxyguanylate (dG) residues were then added per end with 46 U of terminal deoxynucleotidyl transferase in the same reaction mixture (38 µl) as described above, except that 0.1 mM dGTP replaced dTTP. After 20 min. at 37°C the mixture was extracted with phenol-CHCl₃, and the DNA was precipitated with ethanol and digested with 35 U of HindIII endonuclease in 50 µl containing 20 mM Tris.HCl (pH 7.4), 7 mM MgCl2, 60 mM NaCl, and 0.1 mg of BSA at 37°C for 4 hr. The small oligo (dG)-tailed linker DNA was purified by agarose gel (1.8%) electrophoresis and recovered as described above.

2) cDNA Library Preparation:

This was carried out as described in Application PCT/US 86/02464, page 58, line 27 to page 61, line 8, except that 3 µg PolyA⁺ RNA from IgE-induced 23B6 cells was used in the cDNA-synthesis step.

Ampicillin was then added to a concentration of 50 micrograms/ml, and the culture was shaken for an additional 10 hrs. at 37°C. Samples were then taken from the culture and frozen down in DMSO for storage. Separately, samples of the culture were plated and colonies were allowed to form. A large number of colonies were randomly selected and expanded in separate cultures. Plasmid DNA from the expanded cultures was extracted and used to transfect COS 7 monkey cells as follows.

One day prior to transfection, approximately 10⁶ COS 7 monkey cells were seeded onto individual 100 mm plates in Dulbecco's modified Eagle medium (DME) containing 10% fetal calf serum and 2 mM glutamine. To perform the transfection, the medium was aspirated from each plate and replaced with 4 ml of DME containing 50 mM Tris.HCl (pH 7.4), 400 µg/ml DEAE-Dextran and 50 µg of the plasmid DNAs to be tested. The plates were incubated for four hours at 37°C, then the DNA-containing medium was removed, and the plates were washed twice with 5 ml of serum-free DME. DME was added back to the plates which were then incubated for an additional 3 hrs at 37°C. The plates were washed once with DME, and then DME containing 4% fetal calf serum, 2 mM glutamine, penicillin and streptomycin was added. The cells were then incubated for 72 hrs at 37°C. The growth medium was collected and evaluated for GIF activity. After about 1000 assays clone 11C6 was isolated which exhibited GIF activity in the IgE-BF switching assay.

Clone 11C6 was then used as a probe to screen a new library from IgE-induced 23B6 cells constructed in pcD as described above. The BamHI fragment of 11C6 containing the GIF coding region was isolated and labeled by nick-translation. Colonies from the new library were screened by colony hybridization and clones TGIF4, TGIF14, and TGIF19 were selected and amplified. Plasmid DNA was isolated from cultures of each and used to transfect COS 7 cells as described above. Supernatants and cell extracts of the transfected COS 7 cells were tested for GIF activity. Table I lists the location (cellular extract or supernatant) of the GIF activity as well as the cDNA insert lengths of the various clones and the approximate molecular weights (determined by gel filtration chromatography) corresponding to GIF activity. GIF activity was determined by IgE-BF switching.

13

### Table I. Transient Expression of GIF Clones in COS 7 Monkey Cells.

| Clone | Size (kilobases) | Location of GIF Activity | Peaks of GIF Activity (kilodaltons) |
|---|---|---|---|
| TGIF4 | 2.8 | cell | 50; 15 |
| TGIF19 | 2.4 | cell | 40; 15 |
| TGIF14 | 2.4 | supernatant | 40; 15 |
| 11C6 | 0.35 | supernatant | 5-6 |

Analysis of restriction endonuclease fragments and sequencing of the four GIF clones indicated that TGIF14, TGIF19, and 11C6 are 5′-truncated versions of TGIF4.

Example II. Demonstration of Human Messenger RNAs Homologous to a Probe Constructed from TGIF4 by RNA Blotting.

An RNA blot analysis of mRNAs from various human cell lines was carried out using standard techniques; e.g. Maniatis et al., pgs. 202-203 (cited above), or Hames and Higgins, eds., pgs. 139-143, Nucleic Acids Hybridization (IRL Press, Washington, D.C., 1985). RNA was extracted from uninduced U-937, HL-60, 166A2, and RPMI 8866 cells by the guanidinium isothiocyanate method as described above. Poly A⁺ mRNA was selected using oligo(dT) paper as described above, and then loaded and electrophoresed on separate lanes of an agarose gel containing formaldehyde; Maniatis et al. (cited above), pg. 202. The electrophoretically separated mRNAs were transferred to a nitrocellulose filter and baked. A radioactive hybridization probe was constructed from TGIF4 by (i) isolating TGIF4 plasmid DNA, (ii) digesting the isolated TGIF4 with BamHI, (iii) separating the cDNA insert from the rest of the digestion products by gel electrophoresis, and (iv) labeling the cDNA insert by nick-translation. All protocols used are disclosed by Maniatis et al. (cited above).

The hybridization probe was applied to the mRNA-containing filter in the following manner. The mRNA-containing filter was placed in a plastic bag containing about 30-40 ml pre-hybridization buffer (50% formamide, $5 \times$ SSC, 50 mM sodium phosphate buffer (pH 6.5), 250 micrograms/ml sonicated denatured salmon sperm DNA, 10X Denhardt's solution), sealed, and incubated with gentle agitation at 42°C for 16 hours. The prehybridization buffer was then replaced with hybridization buffer (4:1 pre-hybridization buffer: 50% (w/v) dextran sulfate solution) and the denatured hybridization probe (specific activity of about $5 \times 10^8$ cpm/microgram and concentration of about 5ng/ml), obtained by boiling in water for 3 minutes followed by cooling on ice. The bag was re-sealed and incubated with gentle agitation at 42°C for about 20 hours, after which the filter was washed by four changes of $2 \times$ SSC containing 0.1% SDS for 15 min. at 50°C. The washed filter was exposed to X-ray film at -70°C using an intensifying screen. Each cell type tested produced mRNAs having high degrees of homology to the mouse cDNA probe constructed from TGIF4.

Example III. Preparation of Human GIF by Screening a cDNA Library Constructed from U-937 Cells with a Hybridization Probe Constructed from TGIF4.

A human cDNA library was constructed in the pcD vector from poly A⁺ mRNA isolated from the human macrophage cell line U-937 (available from the American Type Culture Collection under accession number CRL 1593). Procedures for isolating poly A⁺ mRNA, constructing the library in pcD, and amplifying in E. coli MC1061 were the same as those described in Example I.

A clone capable of expressing human GIF was obtained as follows. 10 cm agar plates were inoculated with samples of the library culture so that about 5000 colonies formed per plate. Replicas of the colonies on the agar plates were transferred to nitrocellulose filters for screening by colony hybridization using standard protocols; e.g. Hames and Higgins, pgs. 114-122 (cited above), or Maniatis et al., pgs. 312-319 (cited above). The replicas on nitrocellulose filters were placed on fresh agar plates and incubated until colonies formed. The filters were then removed and placed on a stack of three sheets of 3MM paper (Whatman) saturated with denaturation solution (1.5M NaCl, 0.5M NaOH).

Next, the filters were transferred to a stack of 3MM paper saturated with neutralization buffer (1.5M NaCl, 1M Tris-HCl, pH 7.5) for 5 minutes, after which the filter was transferred to a stack of 3MM paper saturated with $4 \times$ SET buffer ($20 \times$ SET buffer stock has the composition: 3M NaCl, 2mM EDTA, 0.4M Tris-HCl, pH 7.8). After soaking in $4 \times$ SET, the filters were placed on dry 3MM paper and left at room temperature or at 37°C until dry.

The dry filters were then baked at 80°C for 2 hours.

A hybridization probe prepared from TGIF4 as described above was applied to the filters as follows. The filters were soaked in $4\times$ SET for 15 minutes at room temperature, then placed in a polythene freezer bag with 1 ml of pre-hybridization solution ($4\times$ SET, $10\times$ Denhardt's solution, 0.1% SDS, 0.1% sodium pyrophosphate, 50 microgram/ml denatured salmon sperm DNA) per 10 cm² of filter paper, sealed, and incubated at 68°C with gentle agitation for about 2-16 hours. Next, the denatured probe ($10^5$-$10^6$ cpm/ml at specific activity of $5\times10^7$-$5\times10^8$ cpm/microgram) was added to the bag, and the bag was resealed and incubated at 68°C with gentle agitation for about 16-24 hours. The filters were then removed from the bag, washed several times in pre-hybridization solution without denatured salmon sperm DNA, dried, and exposed to X-ray film. The developed film was examined to identify replicate colonies ("positive clones") which hybridized to the probe.

$100\times$ Denhardt's solution contains 2% Ficoll (mol. wt. 400,000), 2% polyvinyl pyrrolidone (mol. wt. 400,000), and 2% bovine serum albumin.

Colonies on the master agar plates corresponding to the positive clones of the replicas were picked and separately amplified. Plasmid DNA was isolated from the amplified cultures and used to transfect COS 7 cells as described above. The supernatants and/or cell extracts of the COS 7 cultures were harvested and assayed for GIF activity.

Example IV. Purification of GIF from the Supernatants of 23A4 Cells and HL-60 Cells.

Rat-mouse T-cell hybridoma 23A4 (described by Huff et al., J. Immunol., Vol. 124, pgs. 509-514 (1982)) or human cell line HL-60, were cultured in Dulbecco's modified Eagle's medium containing 5% fetal calf serum. Culture supernatant was recovered three to four days after subculture. In the case of HL-60 cells, phenyl methyl sulfonyl fluoride (PMSF) was added to a final concentration of 1 mM, to inactivate proteases present in the culture supernatant. Supernatants were filtered through Diaflo membrane XM 50 (cut-off point MW 50,000) or XM 100 (cut-off MW 100,000). Filtrates were concentrated 100 to 200 fold by ultrafiltration using YM 5 membranes. Concentrated filtrate was contacted with anti-lipomodulin antibody (produced by hybridoma 141B9, deposited with the American Type Culture Collection, Rockville, Maryland, under accession number HB 8918) immobilized on agarose beads to absorb GIF. The agarose beads were then washed with Dulbecco's phosphate buffered saline (DPBS), and the proteins were eluted with 0.1M glycine-HCl buffer, pH 3.0.

All fractions from 23A4 supernatants were dialyzed against phosphate buffered saline, and the distribution of GIF in the fractions was determined by the ability of each fraction to switch normal BALB/c splenocytes to the formation of unglycosylated IgE-binding factors as disclosed below.

Aliquots of BALB/c spleen cells were incubated for 24 hours with 10 µg per ml of mouse IgE in the presence of each fraction. IgE-binding factors were purified using IgE coupled to agarose beads. IgE-binding factors formed by the BALB/c spleen cells were fractionated on lentil lectin linked to agarose beads. Lentil lectin binds the mannose-rich oligosaccharide of IgE-potentiating factor but fails to bind IgE-suppressive factor.

Fractionation of IgE-binding factors on lentil lectin agarose (Sepharose obtained from Pharmacia Fine Chemicals, Piscataway, New Jersey) was performed by the procedures described in Yodoi et al., J. Immunol., Vol. 125, pg. 1436 (1980). The culture filtrates were concentrated four-fold and 1 ml of the concentrated preparation was absorbed with 0.5 mls of lentil lectin Sepharose. The effluent and DPBS liquid (3 mls) were pooled, and factors bound to the beads were eluted with 0.2 M alpha-methylmannoside. After dialysis against DPBS, the volumes of the effluent and eluate fractions were adjusted to 4 ml.

The effluent and eluate fractions from the lentil lectin agarose were assayed for biological activity by determining their ability to inhibit rosette formation of Fc-epsilon receptor positive cells. Normal BALB/c splenic lymphocytes were employed as a source of Fc-epsilon receptor positive cells, and erythrocytes coated with rat IgE were used to form rosettes. The procedure for inhibition of rosette formation has been described by Yodoi and Ishizaka, J. Immunol., Vol. 124, pg. 1322 (1980). The percentage of rosette inhibition was determined for duplicate tubes and was expressed as the average. The experimental variation between duplicate tubes was less than $\pm$ 10% of the average. The results are shown below in Table II;

## TABLE II

### Purification of GIF by
### Anti-Lipomodulin Linked Agarose Beads

| Fraction of Culture Filtrate | Dilution | IgE-binding factors | |
| --- | --- | --- | --- |
| | | Unfraction-ated % | Effluent/ Eluate % |
| Unfractionated | 1:1000 | 58 | 27/26 |
| | 1:100 | 60 | 55/6 |
| Anti-lipomodulin linked agarose effluent | 1:10 | 57 | 27/30 |
| | 1:3 | 56 | 28/28 |
| Anti-lipomodulin linked agarose eluate | 1:1000 | 56 | 24/30 |
| | 1:100 | 56 | 52/5 |
| Medium Control | | 58 | 28/29 |

The descriptions of the foregoing embodiments of the invention have been presented for purpose of illustration and description. They are not intended to be exhaustive or to limit the invention to the precise forms disclosed, and obviously many modifications and variations are possible in light of the above teaching. The embodiments were chosen and described in order to best explain the principles of the invention and its practical application to thereby enable others skilled in the art to best utilize the invention in various embodiments and with various modifications as are suited to the particular use contemplated. It is intended that the scope of the invention be defined only by the claims appended hereto.

Applicants have deposited E. coli MC1061 carrying TGIF4 with the American Type Culture Collection, Rockville, MD, USA (ATCC), under accession number 67311.

**Claims**

1. A nucleic acid capable of encoding a polypeptide exhibiting glycosylation inhibiting factor activity, the nucleic acid having a sequence of nucleotides effectively homologous to the nucleotide sequence defined by the formula:

CGT – CAC – TCA – GTA – CCT – ATT – TCA –

AGC – CTT – CCT – GGG – CCC – CCG – AGA –

TAC – AAG – AGT – CAA – AGG – ATG – GTT –

CCT – CCT – GGA – CAA – TTA – CAT – CCC –

TAC – GTT – TGT – CTG – TTC – TGT – TAT –

TTA – CTT – ACT – CAT – TGT – ATG – GCT –

GGG – ACC – AAA – ATA – CAT – GAA – GAA –

CCG – GCA – GCC – GTT – CTC – TTG – CCG –

AGC – ATC – CTG – CAG – TTG – TAT – AAC –

CTT – GGA – CTC – ACC – CTG – CTG – TCT –

CTC – TAC – ATG – TTC – TAT – GAG – TTG –

GTG – ACA – GGT – GTG – TGG – GAA – GGC –

AAA – TAC – AAC – TTT – TTC – TGC – CAG –

GGA – ACA – CGC – AGC – GCG – GGA – GAA –

TCC – GAT – ATG – AAG – ATC – ATC – CGC –

GTC – CTC – TGG – TGG – TAC – TAC – TTC –

TCC – AAA – CTC – ATC – GAA – TTC – ATG –

GAC – ACC – TTT – TTC – TTC – ATC – CTT –

CGC – AAG – AAC – AAC – CAC – CAG – ATC –

ACC – GTG – CTC – CAT – GTC – TAC – CAC –

CAC – GCT – ACC – ATG – CTC – AAC – ATC –

450
TGG - TGG - TTT - GTG - ATG - AAC - TGG -
                                      480
GTT - CCC - TGC - GGC - CAT - TCA - TAT -

TTT - GGT - GCG - ACA - CTC - AAC - AGC -
      510
TTC - ATC - CAT - GTC - CTC - ATG - TAC -
                              540
TCG - TAC - TAT - GGT - CTG - TCC - TCC -

ATC - CCG - TCC - ATG - CGT - CCC - TAC -
570
CTC - TGG - TGG - AAA - AAG - TAC - ATC -
                  600
ACT - CAA - GGG - CAG - CTG - GTC - CAG -
                                      630
TTT - GTG - CTG - ACA - ATC - ATC - CAG -

ACG - ACC - TGC - GGG - GTC - TTC - TGG -
      660
CCA - TGC - TCC - TTC - CCT - CTC - GGG -
                              690
TGG - CTG - TTC - TTC - CAG - ATT - GGA -

TAC - ATG - ATT - TCC - CTG - ATT - GCT -
      720
CTC - TTC - ACA - AAC - TTC - TAC - ATT -
                        750
CAG - ACT - TAC - AAC - AAG - AAA - GGG -

GCC - TCT - CGG - AGG - AAA - GAA - CAC -
780
CTG - AAG - GGC - CAC - CAG - AAC - GGG -
                  810
TCT - GTG - GCC - GCC - GTC - AAC - GGA -
                                      840
CAC - ACC - AAC - AGC - TTC - CCT - TCC -

CTG - GAA - AAC - AGC - GTG - AAG - CCC -
            870
AGG - AAG - CAG - CGA - AAG - GAT - TGA -
                                    900
CAA - GCC - GAG - CCG - AAG - CCT - CCA -

GAC - CGC - GGC - GTG - TGA - TTG - TAA -
      930
GCA - CAG - CGT - GAC - TTG - TGC - CCT -
                        960
GAC - GCT - CAT - AGC - AGC - TGC - TGT -

CAC - TAG - TCT - GGC - CCT - ACT - ATC -

```
990
TGC - AGT - GTG - ACT - CAT - GTA - GGA -
                 1020
CCT - TCT - TCA - TCA - GTT - CAA - AAC -
                                     1050
CCC - TAG - AAT - ATG - TAT - ACA - GAT -

TAT - ACA - AAG - TAG - GCA - TAC - GAT -
                 1080
TTT - CTC - ACA - TTT - CTG - GGC - TCT -
                                 1110
CAT - CGA - CCC - CCG - CGC - TAG - ACT -

GTG - GAA - AGG - GAA - TGT - TCT - TGT -
       1140
AGT - ATA - CAA - CAC - TGC - TGT - TGC -
                         1170
CTT - ATT - AGT - TGT - AAC - ACG - ATA -

GGT - GCT - GAA - TTG - TGG - TTC - ACA -
1200
ATT - TAA - AAC - ACT - GTA - ATC - CAA -
                 1230
ACT - TTT - TTT - TTT - TAA - CTG - TAG -
                                 1260
ACC - ATG - CAT - GTG - ATT - GTA - AAT -

TTG - TAC - AAT - GTT - GTT - AAC - AGT -
                 1290
AGA - AAA - ACA - CAC - ATG - CCT - TAA -
                             1320
GAC - TTA - AAA - AAG - CAG - GGC - CCA -

GAG - CTT - GTT - AGT - TTA - ATT - AGG -
       1350
GTA - TGT - TTC - AAC - TTT - GTA - TTA -
                         1380
TTC - ATT - TGT - AAT - AGC - TCT - GTT -

TAG - AAA - AAT - CAA - ATG - TAT - GAT -
1410
TTA - TGA - AAC - AAG - TGT - GTG - ATG -
                 1440
TGT - AAC - TTC - AAG - TGA - CTT - GGA -
                                 1470
GAT - GCG - AAA - TCT - GAT - GGC - ACC -

CTT - GGT - TTT - ATA - ACG - ATG - GCT -
                 1500
TTA - AAT - AAG - CAG - GCT - CAA - GTC -
```

TAG - TGG - AAC - AGT - CAG - TTT - AAC -

TTT - TTA - ACA - GAT - CTT - ATT - TTT -

TTA - TTT - TGA - GTG - CCA - CTA - TTA -

ATG - TGT - TTA - AAG - AGG - TGG - GGA -

AAG - GGT - CCA - AAG - CGT - CTG - GAG -

ACA - CTT - ACG - TAC - CTG - TCC - CTG -

GTC - CCC - CAG - AGG - CTA - GGG - TGA -

CTA - TAA - GAG - TAG - GTC - ACT - TTT -

CAC - TTC - TGA - AGT - GCT - TAG - TGT -

TTT - TAT - ACA - GGA - GAT - AAA - AAG -

GCC - ATT - CGA - CTT - CCC - ACC - ACT -

TGG - AGA - GAG - AGA - ACT - ACC - TTT -

CAG - GAA - AGG - TGA - CGA - GAC - ATT -

TAT - TAT - CTG - ACA - GAC - ACA - CAC -

ACA - CAC - ACA - CAC - AGT - GAT - AAG -

GGA - CTG - GTT - ACC - TGG - GTC - CAC -

ATT - TTT - TTT - TAT - TCT - TTT - CTG -

TAG - TGC - TAC - TTT - GAC - ATA - CAC -

TTC - CTT - TTA - AAA - TGT - GCC - TAA -

TTT - ACC - AAG - AGA - CCA - GGA - AGC -

ACC - ATA - AGC - ATT - TTG - AGG - TAA -

AAT - TTA - AGA - GCT - GGA - GCG - CGC -

AGG - AAC - TGG - GTG - TTT - GGG - AGC -

AAC - CGC - TTT - TTT - CAG - TGT - GAA -

TCA - TGG - TGG - CAT - CTG - TAA - TTT -

TTT - TCT - GAT - TAG - CAC - CTA - AAG -

```
                   2070
ATC - GGT - TTA - AAG - TGC - TTG - GAC -
                                  2100
TGC - TTA - TCC - TAA - AAT - CGT - GTC -

TAG - TGC - GCT - CGA - GGC - ATC - GGT -
            2130
CAT - ACA - AAT - GGG - GAC - TCT - TTT -
                            2160
CAT - GTG - TGT - CAT - TTT - TAA - AGT -

GTT - GAA - AGC - TTA - GCC - TTT - GAA -
      2190
TAC - CTT - CTG - CAT - TGG - TAA - AAA -
                  2220
ATC - CAT - GGA - ACC - ACT - CAT - GGC -

ACA - TCT - TAG - GAG - ATG - ATT - GAC -
2250
AAT - GTA - TAA - ACT - AAT - TGT - GGG -
                  2280
TTT - GAT - ATT - TAT - GTA - AAT - ACC -
                                  2310
AGT - TTA - CCA - TGC - TTT - AAT - TTT -

GCA - CAT - TCA - TAT - TAC - AAG - GAG -
            2340
CCA - GTT - GGT - CCT - AAT - TAG - CCT -
                            2370
TGT - GGG - TTT - TCT - GTT - TGG - AAG -

GAG - TTG - CGG - CAT - CTG - TTT - ACA -
      2400
TTT - CCC - TTG - TCA - GAT - CTA - GAA -
                  2430
TGT - GTG - TAT - GTG - TGT - GTC - TTC -

TCC - CTA - GGT - ACC - CGT - CTG - CAG -
      2460
GTG - TCT - TTA - CAT - ATC - TCA - ATA -
                  2490
CAA - AAA - TTA - TAA - CAT - TTG - ATA -

GTG - TGC - TGT - CAA - AGT - GTG - CTT -
2520
AGC - TCA - TCT - GGA - TAT - ACC - CAC -
                  2550
ACT - GTT - AAA - TAA - TGT - CTA - AAC -
                            2580
ATT - AAT - CAT - TAA - AAC - CTT - TTT -

GAT - TAA - AAA.
```

2. The nucleic acid of claim 1 wherein said polypeptide exhibits human glycosylation inhibiting factor activity, and wherein said sequence of nucleotides is at least seventy percent (70%) homologous to said nucleotide sequence defined by said formula.

3. The nucleic acid of claim 2 obtained from messenger RNA produced by the human cell line U-937.

4. A method of producing a polypeptide exhibiting human glycosylation inhibiting factor activity, the method comprising the steps of:

(a) constructing a vector comprising a nucleotide sequence coding for the polypeptide, wherein the nucleotide sequence is capable of being expressed by a host containing the vector and wherein the nucleotide sequence is effectively homologous to the sequence defined by the formula:

```
CGT - CAC - TCA - GTA - CCT - ATT - TCA -
                30
AGC - CTT - CCT - GGG - CCC - CCG - AGA -
                                  60
TAC - AAG - AGT - CAA - AGG - ATG - GTT -

CCT - CCT - GGA - CAA - TTA - CAT - CCC -
 90
TAC - GTT - TGT - CTG - TTC - TGT - TAT -
                        120
TTA - CTT - ACT - CAT - TGT - ATG - GCT -

GGG - ACC - AAA - ATA - CAT - GAA - GAA -
150
CCG - GCA - GCC - GTT - CTC - TTG - CCG -
                  180
AGC - ATC - CTG - CAG - TTG - TAT - AAC -
                                  210
CTT - GGA - CTC - ACC - CTG - CTG - TCT -

CTC - TAC - ATG - TTC - TAT - GAG - TTG -
            240
GTG - ACA - GGT - GTG - TGG - GAA - GGC -
```

```
                                        270
AAA - TAC - AAC - TTT - TTC - TGC - CAG -

GGA - ACA - CGC - AGC - GCG - GGA - GAA -
      300
TCC - GAT - ATG - AAG - ATC - ATC - CGC -
                              330
GTC - CTC - TGG - TGG - TAC - TAC - TTC -

TCC - AAA - CTC - ATC - GAA - TTC - ATG -
360
GAC - ACC - TTT - TTC - TTC - ATC - CTT -
            390
CGC - AAG - AAC - AAC - CAC - CAG - ATC -
                                    420
ACC - GTG - CTC - CAT - GTC - TAC - CAC -

CAC - GCT - ACC - ATG - CTC - AAC - ATC -
            450
TGG - TGG - TTT - GTG - ATG - AAC - TGG -
                              480
GTT - CCC - TGC - GGC - CAT - TCA - TAT -

TTT - GGT - GCG - ACA - CTC - AAC - AGC -
      510
TTC - ATC - CAT - GTC - CTC - ATG - TAC -
                        540
TCG - TAC - TAT - GGT - CTG - TCC - TCC -

ATC - CCG - TCC - ATG - CGT - CCC - TAC -
570
CTC - TGG - TGG - AAA - AAG - TAC - ATC -
                  600
ACT - CAA - GGG - CAG - CTG - GTC - CAG -
                                    630
TTT - GTG - CTG - ACA - ATC - ATC - CAG -

ACG - ACC - TGC - GGG - GTC - TTC - TGG -
            660
CCA - TGC - TCC - TTC - CCT - CTC - GGG -
                              690
TGG - CTG - TTC - TTC - CAG - ATT - GGA -

TAC - ATG - ATT - TCC - CTG - ATT - GCT -
      720
CTC - TTC - ACA - AAC - TTC - TAC - ATT -
                        750
CAG - ACT - TAC - AAC - AAG - AAA - GGG -

GCC - TCT - CGG - AGG - AAA - GAA - CAC -
780
CTG - AAG - GGC - CAC - CAG - AAC - GGG -
```

```
                    810
TCT - GTG - GCC - GCC - GTC - AAC - GGA -
                                    840
CAC - ACC - AAC - AGC - TTC - CCT - TCC -

CTG - GAA - AAC - AGC - GTG - AAG - CCC -
            870
AGG - AAG - CAG - CGA - AAG - GAT - TGA -
                                    900
CAA - GCC - GAG - CCG - AAG - CCT - CCA -

GAC - CGC - GGC - GTG - TGA - TTG - TAA -
            930
GCA - CAG - CGT - GAC - TTG - TGC - CCT -
                        960
GAC - GCT - CAT - AGC - AGC - TGC - TGT -

CAC - TAG - TCT - GGC - CCT - ACT - ATC -
990
TGC - AGT - GTG - ACT - CAT - GTA - GGA -
                  1020
CCT - TCT - TCA - TCA - GTT - CAA - AAC -
                                    1050
CCC - TAG - AAT - ATG - TAT - ACA - GAT -

TAT - ACA - AAG - TAG - GCA - TAC - GAT -
            1080
TTT - CTC - ACA - TTT - CTG - GGC - TCT -
                              1110
CAT - CGA - CCC - CCG - CGC - TAG - ACT -

GTG - GAA - AGG - GAA - TGT - TCT - TGT -
      1140
AGT - ATA - CAA - CAC - TGC - TGT - TGC -
                        1170
CTT - ATT - AGT - TGT - AAC - ACG - ATA -

GGT - GCT - GAA - TTG - TGG - TTC - ACA -
1200
ATT - TAA - AAC - ACT - GTA - ATC - CAA -
                  1230
ACT - TTT - TTT - TTT - TAA - CTG - TAG -
                                    1260
ACC - ATG - CAT - GTG - ATT - GTA - AAT -

TTG - TAC - AAT - GTT - GTT - AAC - AGT -
                  1290
AGA - AAA - ACA - CAC - ATG - CCT - TAA -
                              1320
GAC - TTA - AAA - AAG - CAG - GGC - CCA -

GAG - CTT - GTT - AGT - TTA - ATT - AGG -
```

24

```
GTA - TGT - TTC - AAC - TTT - GTA - TTA -

TTC - ATT - TGT - AAT - AGC - TCT - GTT -

TAG - AAA - AAT - CAA - ATG - TAT - GAT -

TTA - TGA - AAC - AAG - TGT - GTG - ATG -

TGT - AAC - TTC - AAG - TGA - CTT - GGA -

GAT - GCG - AAA - TCT - GAT - GGC - ACC -

CTT - GGT - TTT - ATA - ACG - ATG - GCT -

TTA - AAT - AAG - CAG - GCT - CAA - GTC -

TAG - TGG - AAC - AGT - CAG - TTT - AAC -

TTT - TTA - ACA - GAT - CTT - ATT - TTT -

TTA - TTT - TGA - GTG - CCA - CTA - TTA -

ATG - TGT - TTA - AAG - AGG - TGG - GGA -

AAG - GGT - CCA - AAG - CGT - CTG - GAG -

ACA - CTT - ACG - TAC - CTG - TCC - CTG -

GTC - CCC - CAG - AGG - CTA - GGG - TGA -

CTA - TAA - GAG - TAG - GTC - ACT - TTT -

CAC - TTC - TGA - AGT - GCT - TAG - TGT -

TTT - TAT - ACA - GGA - GAT - AAA - AAG -

GCC - ATT - CGA - CTT - CCC - ACC - ACT -

TGG - AGA - GAG - AGA - ACT - ACC - TTT -

CAG - GAA - AGG - TGA - CGA - GAC - ATT -

TAT - TAT - CTG - ACA - GAC - ACA - CAC -

ACA - CAC - ACA - CAC - AGT - GAT - AAG -

GGA - CTG - GTT - ACC - TGG - GTC - CAC -

ATT - TTT - TTT - TAT - TCT - TTT - CTG -

TAG - TGC - TAC - TTT - GAC - ATA - CAC -
```

TTC – CTT – TTA – AAA – TGT – GCC – TAA –
                    1920
TTT – ACC – AAG – AGA – CCA – GGA – AGC –
                                    1950
ACC – ATA – AGC – ATT – TTG – AGG – TAA –

AAT – TTA – AGA – GCT – GGA – GCG – CGC –
          1980
AGG – AAC – TGG – GTG – TTT – GGG – AGC –
                          2010
AAC – CGC – TTT – TTT – CAG – TGT – GAA –

TCA – TGG – TGG – CAT – CTG – TAA – TTT –
2040
TTT – TCT – GAT – TAG – CAC – CTA – AAG –
                    2070
ATC – GGT – TTA – AAG – TGC – TTG – GAC –
                                      2100
TGC – TTA – TCC – TAA – AAT – CGT – GTC –

TAG – TGC – GCT – CGA – GGC – ATC – GGT –
          2130
CAT – ACA – AAT – GGG – GAC – TCT – TTT –
                                2160
CAT – GTG – TGT – CAT – TTT – TAA – AGT –

GTT – GAA – AGC – TTA – GCC – TTT – GAA –
          2190
TAC – CTT – CTG – CAT – TGG – TAA – AAA –
                          2220
ATC – CAT – GGA – ACC – ACT – CAT – GGC –

ACA – TCT – TAG – GAG – ATG – ATT – GAC –
2250
AAT – GTA – TAA – ACT – AAT – TGT – GGG –
                    2280
TTT – GAT – ATT – TAT – GTA – AAT – ACC –
                                      2310
AGT – TTA – CCA – TGC – TTT – AAT – TTT –

GCA – CAT – TCA – TAT – TAC – AAG – GAG –
          2340
CCA – GTT – GGT – CCT – AAT – TAG – CCT –
                                2370
TGT – GGG – TTT – TCT – GTT – TGG – AAG –

GAG – TTG – CGG – CAT – CTG – TTT – ACA –
          2400
TTT – CCC – TTG – TCA – GAT – CTA – GAA –
                          2430
TGT – GTG – TAT – GTG – TGT – GTC – TTC –

```
TCC  -  CTA  -  GGT  -  ACC  -  CGT  -  CTG  -  CAG  -
       2460
GTG  -  TCT  -  TTA  -  CAT  -  ATC  -  TCA  -  ATA  -
                            2490
CAA  -  AAA  -  TTA  -  TAA  -  CAT  -  TTG  -  ATA  -

GTG  -  TGC  -  TGT  -  CAA  -  AGT  -  GTG  -  CTT  -
2520
AGC  -  TCA  -  TCT  -  GGA  -  TAT  -  ACC  -  CAC  -
                    2550
ACT  -  GTT  -  AAA  -  TAA  -  TGT  -  CTA  -  AAC  -
                                          2580
ATT  -  AAT  -  CAT  -  TAA  -  AAC  -  CTT  -  TTT  -

GAT  -  TAA  -  AAA;
```

(b) incorporating the vector into the host; and

(c) maintaining the host in a culture medium under conditions suitable for expression of the nucleotide sequence into said polypeptide.

5. The method of claim 4 further comprising the step of separating said polypeptide from said culture medium and said host.

6. The method of claim 4 wherein said vector is a pcD vector and said host is selected from the group consisting of COS monkey cells, Chinese hamster ovary cells, and mouse L cells.

7. The vector TGIF4 deposited with the American Type Culture Collection under accession number 67311.

0285405

FIGURE 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y,D | THE JOURNAL OF IMMUNOLOGY, vol. 130, no. 2, February 1983, pages 878-884, The American Association of Immunologists, Maryland, US; T. UEDE et al.: "Modulation of the biologic activities of IgE-binding factors" * Page 883, column 1, lines 34-36 * | 1,2,4-6 | C 12 N 15/00 C 12 P 21/02 C 07 K 13/00 // A 61 K 37/64 |
| Y | EP-A-0 155 192 (SCHERING BIOTECH CORP. & THE JOHN HOPKINS UNIVERSITY) * Whole document * | 1,2,4-6 | |
| A,D | WO-A-8 604 094 (BIOGEN N.V. et al.) * Page 3, lines 14-23 * | | |
| A,D | ANNUAL REVIEWS OF IMMUNOLOGY, 1984, Annual Reviews Inc., Palo Alto, California, US; K. ISHIZAKA: "Regulation of IgE synthesis" * Page 174, line 32 - page 175, line 18 * | | |

TECHNICAL FIELDS
SEARCHED (Int. Cl.4)

C 12 N
C 12 P

The present search report has been drawn up for all claims

| Place of search | Date of completion of the search | Examiner |
|---|---|---|
| THE HAGUE | 26-06-1988 | VAN PUTTEN A.J. |

EPO FORM 1503 03.82 (P0401)